# EUROPEAN PATENT APPLICATION

(11) **EP 1 683 795 A1**
(43) Date of publication of application: **26.07.2006**
(21) Application number: 05100400.0
(22) Date of filing: 21.01.2005
(51) Int. Cl.: C07D 409/12, A61K 31/4436, A61P 11/00, A61P 29/00

(54) **Crystalline forms of cis-5-fluoro-N-¬4-(2-hydroxy-4-methylbenzamido)cyclohexyl|-2-(tetrahydrothiopyran-4-yloxy)nicotinamide**

(71) Applicant: Pfizer Limited, Sandwich, Kent CT13 9NJ (GB)
(72) Inventor: The designation of the inventor has not yet been filed
(74) Representative: Laurent, Claire

(57) **Abstract**

The present invention relates to new crystalline forms of syn-5-Fluoro-N-[4-(2-hydroxy-4-methyl-benzoylamino)-cyclohexyl]-2-(tetrahydro-thiopyran-4-yloxy)-nicotinamide and to processes for the preparation of, compositions containing and the uses of such crystalline forms.

## Description

The present invention relates to new crystalline forms of cis-5-Fluoro-N-[4-(2-hydroxy-4-methylbenzamido)cyclohexyl]-2-(tetrahydro-thiopyran-4-yloxy)-nicotinamide and salts thereof, to a process for the preparation of, compositions containing and the uses of such crystalline forms.

*Cis*-5-Fluoro-N-[4-(2-hydroxy-4-methylbenzamido)cyclohexyl]-2-(tetrahydrothiopyran-4-yloxy)nicotinamide (also known as Syn-5-Fluoro-N-[4-(2-hydroxy-4-methyl-benzoylamino)-cyclohexyl]-2-(tetrahydro-thiopyran-4-yloxy)-nicotinamide) has the structure shown in formula (I) : 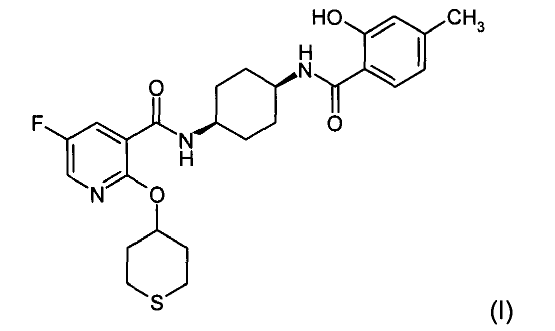 and its preparation is disclosed in the International Patent Application number PCT/IB04/002367 (not published yet).

As described in PCT/IB04/002367, *cis*-5-Fluoro-N-[4-(2-hydroxy-4-methylbenzamido)cyclohexyl]-2-(tetrahydro-thiopyran-4-yloxy)-nicotinamide is a PDE4 inhibitor and may be used in the treatment of various inflammatory allergic and respiratory diseases and conditions. It may therefore be used to treat any disease for which a PDE4 inhibitor is indicated such as for example, but not exclusively, adult respiratory distress syndrome (ARDS), bronchitis, chronic bronchitis, chronic obstructive pulmonary disease (COPD), cystic fibrosis, asthma, emphysema, bronchiectasis, chronic sinusitis, rhinitis, inflammatory bowel diseases (IBD) such as Crohn's disease, ileitis, collagenous colitis, colitis polyposa, transmural colitis and ulcerative colitis.

Examples 63 of PCT/IB04/002367 describes the preparation of cis-5-Fluoro-N-[4-(2-hydroxy-4-methylbenzamido)cyclohexyl]-2-(tetrahydro-thiopyran-4-yloxy)-nicotinamide. Content of example 63 of PCT/IB04/002367 is given in the Example section. This preparation provides a solid form of said compound (here after named "Form A").

Before a drug compound can be commercialised, a process for its bulk manufacture must be developed that reliably provides a uniform and highly pure grade of the compound. Further, the process must deliver a form of the compound that can be suitably formulated for convenient dosage to patients and which is chemically and physically stable over long periods in that formulation.

Crystalline forms of a drug compound have advantages in several respects. For example, the compound can be easily purified by crystallisation and recrystallisation. Crystallisation is a much cheaper and more convenient method of purification to perform on a large scale than other known methods of purification such as chromatography. Further, a crystalline form is usually more stable than any other form (amorphous form), both before and during formulation and during subsequent storage. Finally, when formulating a drug for delivery by inhalation, it is generally easier to mill or micronise a crystalline form to a respirable size (generally considered as particles less than 5 microns in diameter) than an amorphous form. Thus, once a drug compound has been identified, there is a huge need for further identification of its crystalline forms.

There are no generally applicable methods for preparing crystalline forms. Indeed, it is impossible to know, from the outset, whether any crystalline form of a given compound exists. Where it turns out that a compound can be crystallised, extensive experimentation is usually required before a process is identified from which the crystalline forms can be isolated. The correct combination of several independently variable conditions (for example, solvent concentration, solvent composition, temperature, cooling rate) must be identified empirically through trial and error with no guarantee of success.

It has now been found that several crystalline forms of cis-5-Fluoro-N-[4-(2-hydroxy-4-methylbenzamido)cyclohexyl]-2-(tetrahydro-thiopyran-4-yloxy)-nicotinamide exist and may be prepared using the processes outlined below in the example section.

The invention thus provides crystalline forms of *cis*-5-Fluoro-N-[4-(2-hydroxy-4-methylbenzamido)cyclohexyl]-2-(tetrahydro-thiopyran-4-yloxy)-nicotinamide with the proviso of Form A.

According to a further aspect, the present invention provides anhydrous, monohydrate and solvate crystalline forms of *cis*-5-Fluoro-N-[4-(2-hydroxy-4-methylbenzamido)cyclohexyl]-2-(tetrahydro-thiopyran-4-yloxy)-nicotinamide with the proviso of Form A.

According to a further aspect, the present invention provides forms B, C, D and F of *cis*-5-Fluoro-N-[4-(2-hydroxy-4-methylbenzamido)cyclohexyl]-2-(tetrahydrothiopyran-4-yloxy)-nicotinamide as further characterized in the example section below.

According to a final aspect of the present invention, the crystalline form C as further characterized in the example section below is preferred.

Pharmaceutically acceptable salts of the crystalline forms of cis-5-Fluoro-N-[4-(2-hydroxy-4-methylbenzamido)cyclohexyl]-2-(tetrahydro-thiopyran-4-yloxy)-nicotinamide according to the present invention (henceforth referred to as 'the compounds of the invention') include the acid addition and base salts thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of the compound of the invention may be prepared by one or more of three methods:
(i) by reacting the crystalline forms of the present invention with the desired acid or base;
(ii) by removing an acid- or base-labile protecting group from a suitable precursor of the crystalline forms of the present invention or by ring-opening a suitable cyclic precursor, for example, a lactone or lactam, using the desired acid or base; or
(iii) by converting one salt of the crystalline forms of the present invention to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention and salts thereof may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

The compounds of the invention and salts thereof may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention and salts thereof may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula I, a film-forming polymer, a binder, a solvent, a humectant, a plasticiser, a stabiliser or emulsifier, a viscosity-modifying agent and a solvent. Some components of the formulation may perform more than one function.

The compound of the invention and salts thereof may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of the invention and salts thereof may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, cosolvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma *et al* (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of the invention and salts thereof used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a suspension or as a solid, semi-solid, or thixotropic liquid for administration as an implanted depot providing modified release of the active compound. Examples of such formulations include drug-coated stents and semi-solids and suspensions comprising drug-loaded poly(*dl*-lactic-coglycolic)acid (PGLA) microspheres.

The compounds of the invention may also be administered topically, (intra)dermally, or transdermally to the skin or mucosa. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention and salts thereof can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula I, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 1 µg to 4000 µg of the compound of formula I. The overall daily dose will typically be in the range 1 µg to 20 mg which may be administered in a single dose or, more usually, as divided doses throughout the day.

The compounds of the invention and salts thereof may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention and salts thereof may also be administered directly to the eye or ear, typically in the form of drops of a micronised suspension or solution in isotonic, pH-adjusted, sterile saline. Other formulations suitable for ocular and aural administration include ointments, gels, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.
Formulations for ocular/aural administration may be formulated to be immediate and/or modified release. Modified release formulations include delayed-, sustained-, pulsed-, controlled-, targeted, or programmed release.

The compounds of the invention and salts thereof may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e.* as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula I in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range of 0.001 mg/kg to 100 mg/kg depending, of course, on the mode of administration. The total daily dose may be administered in single or divided doses and may, at the physician's discretion, fall outside of the typical range given herein.

These dosages are based on an average human subject having a weight of about 60kg to 70kg. The physician will readily be able to determine doses for subjects whose weight falls outside this range, such as infants and the elderly. For the avoidance of doubt, references herein to "treatment" include references to curative, palliative and prophylactic treatment.

The crystalline forms of *cis*-5-Fluoro-N-[4-(2-hydroxy-4-methylbenzamido)cyclohexyl]-2-(tetrahydro-thiopyran-4-yloxy)-nicotinamide provided by the present invention and salts thereof may optionally be formulated in combination with other pharmacologically active compounds. Preferred combinations for use in the treatment of obstructive airways and other inflammatory diseases include combinations with:
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1, H3 and H4 antagonists,
(d) α₁- and α₂-adrenoceptor agonist vasoconstrictor sympathomimetic agents for decongestant use,
(e) muscarinic M3 receptor antagonists or anticholinergic agents,
(f) β2-adrenoceptor agonists,
(g) PDE inhibitors, e.g. PDE3 and PDE5 inhibitors,
(h) Theophylline,
(i) Sodium cromoglycate,
(j) COX inhibitors both non-selective and selective COX-1 or COX-2 inhibitors (NSAIDs),
(k) Oral and inhaled glucocorticosteroids, such as DAGR (dissociated agonists of the corticoid receptor),
(I) Monoclonal antibodies active against endogenous inflammatory entities,
(m)Anti-tumor necrosis factor (anti-TNF-α) agents,
(n) Adhesion molecule inhibitors including VLA-4 antagonists,
(o) Kinin-B₁ - and B₂-receptor antagonists,
(p) Immunosuppressive agents,
(q) Inhibitors of matrix metalloproteases (MMPs),
(r) Tachykinin NK₁, NK₂ and NK₃ receptor antagonists,
(s) Elastase inhibitors,
(t) Adenosine A2a receptor agonists,
(u) Inhibitors of urokinase,
(v) Compounds that act on dopamine receptors, e.g. D2 agonists,
(w)Modulators of the NFκβ pathway, e.g. IKK inhibitors,
(x) modulators of cytokine signalling pathyways such as p38 MAP kinase, syk kinase or JAK kinase inhibitor,
(y) Agents that can be classed as mucolytics or anti-tussive, and
(z) Antibiotics,
(aa) HDAC inhibitors and
(bb) PI3 kinase inhibitors.

It is to be appreciated that all references herein to treatment include curative, palliative and prophylactic treatment. Therefore, a further aspect of the present invention relates to the use of the compounds of the invention and salts thereof in the treatment of diseases, disorders, and conditions in which the PDE4 isozymes are involved. More specifically, the present invention also concerns the use of the compounds of the invention in the treatment of diseases, disorders, and conditions selected from the group consisting of :
■ asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma and wheezy infant syndrome,
■ chronic or acute bronchoconstriction, chronic bronchitis, small airways obstruction, and emphysema,
■ obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated therewith, COPD that is characterized by irreversible, progressive airways obstruction, adult respiratory distress syndrome (ARDS) and exacerbation of airways hyper-reactivity consequent to other drug therapy
■ pneumoconiosis of whatever type, etiology, or pathogenesis, in particular pneumoconiosis that is a member selected from the group consisting of aluminosis or bauxite workers' disease, anthracosis or miners' asthma, asbestosis or steam-fitters' asthma, chalicosis or flint disease, ptilosis caused by inhaling the dust from ostrich feathers, siderosis caused by the inhalation of iron particles, silicosis or grinders' disease, byssinosis or cotton-dust asthma and talc pneumoconiosis;
■ bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
■ bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis,
■ seasonal allergic rhinitis or perennial allergic rhinitis or sinusitis of whatever type, etiology, or pathogenesis, in particular sinusitis that is a member selected from the group consisting of purulent or nonpurulent sinusitis, acute or chronic sinusitis and ethmoid, frontal, maxillary, or sphenoid sinusitis,
■ rheumatoid arthritis of whatever type, etiology, or pathogenesis, in particular rheumatoid arthritis that is a member selected from the group consisting of acute arthritis, acute gouty arthritis, chronic inflammatory arthritis, degenerative arthritis, infectious arthritis, Lyme arthritis, proliferative arthritis, psoriatic arthritis and vertebral arthritis,
■ gout, and fever and pain associated with inflammation,
■ an eosinophil-related disorder of whatever type, etiology, or pathogenesis, in particular an eosinophil-related disorder that is a member selected from the group consisting of eosinophilia, pulmonary infiltration eosinophilia, Loffler's syndrome, chronic eosinophilic pneumonia, tropical pulmonary eosinophilia, bronchopneumonic aspergillosis, aspergilloma, granulomas containing eosinophils, allergic granulomatous angiitis or Churg-Strauss syndrome, polyarteritis nodosa (PAN) and systemic necrotizing vasculitis,
■ atopic dermatitis, allergic dermatitis, contact dermatitis, or allergic or atopic eczema,
■ urticaria of whatever type, etiology, or pathogenesis, in particular urticaria that is a member selected from the group consisting of immune-mediated urticaria, complement-mediated urticaria, urticariogenic material-induced urticaria, physical agent-induced urticaria, stress-induced urticaria, idiopathic urticaria, acute urticaria, chronic urticaria, angioedema, cholinergic urticaria, cold urticaria in the autosomal dominant form or in the acquired form, contact urticaria, giant urticaria and papular urticaria,
■ conjunctivitis of whatever type, etiology, or pathogenesis, in particular conjunctivitis that is a member selected from the group consisting of actinic conjunctivitis, acute catarrhal conjunctivitis, acute contagious conjunctivitis, allergic conjunctivitis, atopic conjunctivitis, chronic catarrhal conjunctivitis, purulent conjunctivitis and vernal conjunctivitis,
■ uveitis of whatever type, etiology, or pathogenesis, in particular uveitis that is a member selected from the group consisting of inflammation of all or part of the uvea, anterior uveitis, iritis, cyclitis, iridocyclitis, granulomatous uveitis, nongranulomatous uveitis, phacoantigenic uveitis, posterior uveitis, choroiditis; and chorioretinitis,
■ psoriasis;
■ multiple sclerosis of whatever type, etiology, or pathogenesis, in particular multiple sclerosis that is a member selected from the group consisting of primary progressive multiple sclerosis and relapsing remitting multiple sclerosis,
■ autoimmune/inflammatory diseases of whatever type, etiology, or pathogenesis, in particular an autoimmune/inflammatory disease that is a member selected from the group consisting of autoimmune hematological disorders, hemolytic anemia, aplastic anemia, pure red cell anemia, idiopathic thrombocytopenic purpura, systemic lupus erythematosus, polychondritis, scleroderma, Wegner's granulomatosis, dermatomyositis, chronic active hepatitis, myasthenia gravis, Stevens-Johnson syndrome, idiopathic sprue, autoimmune inflammatory bowel diseases, ulcerative colitis, endocrin opthamopathy, Grave's disease, sarcoidosis, alveolitis, chronic hypersensitivity pneumonitis, primary biliary cirrhosis, juvenile diabetes or diabetes mellitus type I, keratoconjunctivitis sicca, epidemic keratoconjunctivitis, diffuse interstitial pulmonary fibrosis or interstitial lung fibrosis, idiopathic pulmonary fibrosis, cystic fibrosis, glomerulonephritis with and without nephrotic syndrome, acute glomerulonephritis, idiopathic nephrotic syndrome, minimal change nephropathy, inflammatory/hyperproliferative skin diseases, benign familial pemphigus, pemphigus erythematosus, pemphigus foliaceus, and pemphigus vulgaris,
■ prevention of allogeneic graft rejection following organ transplantation,
■ inflammatory bowel disease (IBD) of whatever type, etiology, or pathogenesis, in particular inflammatory bowel disease that is a member selected from the group consisting of collagenous colitis, colitis polyposa, transmural colitis, ulcerative colitis and Crohn's disease (CD),
■ septic shock of whatever type, etiology, or pathogenesis, in particular septic shock that is a member selected from the group consisting of renal failure, acute renal failure, cachexia, malarial cachexia, hypophysial cachexia, uremic cachexia, cardiac cachexia, cachexia suprarenalis or Addison's disease, cancerous cachexia and cachexia as a consequence of infection by the human immunodeficiency virus (HIV),
■ liver injury,
■ pulmonary hypertension of whatever type, etiology or pathogenesis including primary pulmonary hypertension / essential hypertension, pulmonary hypertension secondary to congestive heart failure, pulmonary hypertension secondary to chronic obstructive pulmonary disease, pulmonary venous hypertension, pulmonary arterial hypertension and hypoxia-induced pulmonary hypertension,
■ bone loss diseases, primary osteoporosis and secondary osteoporosis,
■ central nervous system disorders of whatever type, etiology, or pathogenesis, in particular a central nervous system disorder that is a member selected from the group consisting of depression, Alzheimers disease, Parkinson's disease, learning and memory impairment, tardive dyskinesia, drug dependence, arteriosclerotic dementia and dementias that accompany Huntington's chorea, Wilson's disease, paralysis agitans, and thalamic atrophies,
■ infection, especially infection by viruses wherein such viruses increase the production of TNF-α in their host, or wherein such viruses are sensitive to upregulation of TNF-α in their host so that their replication or other vital activities are adversely impacted, including a virus which is a member selected from the group consisting of HIV-1, HIV-2, and HIV-3, cytomegalovirus (CMV), influenza, adenoviruses and Herpes viruses including *Herpes zoster* and *Herpes simplex,*
■ yeast and fungus infections wherein said yeast and fungi are sensitive to upregulation by TNF-α or elicit TNF-α production in their host, e.g., fungal meningitis, particularly when administered in conjunction with other drugs of choice for the treatment of systemic yeast and fungus infections, including but are not limited to, polymixins, e.g. Polymycin B, imidazoles, e.g. clotrimazole, econazole, miconazole, and ketoconazole, triazoles, e.g. fluconazole and itranazole as well as amphotericins, e.g. Amphotericin B and liposomal Amphotericin B,
■ ischemia-reperfusion injury, ischemic heart disease, autoimmune diabetes, retinal autoimmunity, chronic lymphocytic leukemia, HIV infections, lupus erythematosus, kidney and ureter disease, urogenital and gastrointestinal disorders and prostate diseases,
■ reduction of scar formation in the human or animal body, such as scar formation in the healing of acute wounds, and
■ psoriasis, other dermatological and cosmetic uses, including antiphlogistic, skin-softening, skin elasticity and moisture-increasing activities.
   According to one aspect the present invention relates in particular to the treatment of a respiratory disease, such as adult respiratory distress syndrome (ARDS), bronchitis, chronic bronchitis, chronic obstructive pulmonary disease (COPD), cystic fibrosis, asthma, emphysema, bronchiectasis, chronic sinusitis and rhinitis.

According to another aspect the present invention relates in particular to the treatment of gastrointestinal (GI) disorders, in particular inflammatory bowel diseases (IBD) such as Crohn's disease, ileitis, collagenous colitis, colitis polyposa, transmural colitis and ulcerative colitis.

A still further aspect of the present invention also relates to the use of the compounds according to the present invention for the manufacture of a drug having a PDE4 inhibitory activity. In particular, the present inventions concerns the use of the compounds according to the present invention for the manufacture of a drug for the treatment of inflammatory, respiratory, allergic and scar-forming diseases, disorders, and conditions, and more precisely for the treatment of diseases, disorders, and conditions that are listed above.

As a consequence, the present invention provides a particularly interesting method of treatment of a mammal, including a human being, with a PDE4 inhibitor including treating said mammal with an effective amount of a compound according to the present invention and salts thereof. More precisely, the present invention provides a particularly interesting method of treatment of a mammal, including a human being, to treat an inflammatory, respiratory, allergic and scar-forming disease, disorder or condition, including treating said mammal with an effective amount of a compound according to the present invention.

Further aspects of the invention are mentioned in the claims.

The following Examples illustrate the invention.

### FIGURES

Figure 1: PXRD Pattern for Form C
Figure 2: Calculated PXRD Pattern for Form C
Figure 3: DSC Thermogram for Form C
Figure 4: DSC Thermogram for Form B
Figure 5: PXRD Pattern for Form B
Figure 6: DSC Thermogram for Form D
Figure 7: PXRD Pattern for Form D
Figure 8: DSC Thermogram for Form F
Figure 9: PXRD Pattern for Form F

### PROTOCOLS

For all examples below, the following experimental conditions were used:

### Differential Scanning Calorimetry (DSC)

Differential Scanning Calorimetry was performed using a Perkin Elmer Diamond DSC in aluminium pans with holes and lids. Approximatively 3 mg of the samples were heated at 20°C per minute over ranges of 10°C to 250°C or 10°C to 300°C or 20°C to 300°C depending on the samples, with a nitrogen gas purge.

### Powder X-Ray diffraction (PXRD)

The PXRD patterns were obtained using a Bruker-AXS Ltd. D4 powder X-ray diffractometer fitted with an automatic sample changer, a theta-theta goniometer, automatic beam divergence slits, a secondary monochromator and a scintillation counter. The sample was prepared for analysis by packing the powder into a 12 mm diameter, 0.25 mm deep cavity that had been cut into a silicon wafer specimen mount. The specimen was rotated whilst being irradiated with copper K-alpha₁ X-rays (wavelength = 1.5406 Angstroms) with the X-ray tube operated at 40 kV/40 mA. The analyses were performed with the goniometer running in continuous mode set for a 5 second count per 0.02° step over a two theta range of 2° to 55°.
The peaks obtained for Form C were aligned against those from the calculated pattern from the single crystal structure. For Form F, the peaks obtained were aligned against a silicon reference standard.
For Form C, 2-theta Angles, d spacings and relative intensities were calculated from the single crystal structure using the "Reflex Powder Diffraction" module of Accelrys Materials Studio™ [version 2.2]. Pertinent simulation parameters were in each case:
Wavelength = 1.540562 A (Cu Kα)
Polarisation Factor = 0.5
Pseudo-Voigt Profile (U = 0.01, V = -0.001, W = 0.002)

As will be appreciated by the skilled crystallographer, the relative intensities of the various peaks within Tables given below may vary due to a number of factors such as for example orientation effects of crystals in the X-ray beam or the purity of the material being analysed or the degree of crystallinity of the sample. The peak positions may also shift for variations in sample height but the peak positions will remain substantially as defined in given Tables.

The skilled crystallographer will also appreciate that measurements using a different wavelength will result in different shifts according to the Bragg equation - nλ = 2d sin θ.

Such further PXRD patterns generated by use of alternative wavelengths are considered to be alternative representations of the PXRD patterns of the crystalline materials of the present invention and as such are within the scope of the present invention.

### EXAMPLES

### Example 1: Preparation of Form C (crystalline monohydrate form)

2.45 kg (8.5 ml/g) of isopropyl acetate was charged to the reaction vessel. The following were then added with stirring: 4-methyl salicylic acid (113 g, 0.743 M), the amine of preparation 18 (see below for obtention of the amine of preparation 18 - 290 g 0.746 M), 1-Hydroxybenzotriazole.hydrate (90.3 g, 0.599 M) and triethylamine (151 g, 1.492 M). Reaction was heated to reflux for 2 hours, then cooled to 40°C and 1-(3-dimethylaminopropyl)-3-ethylcarbodiimide hydrochloride (WSCDI) (177 g, 0.923 M) was added. Once added the reaction was heated back to reflux and left to react for 24 hours. In-process check by fast liquid chromatography after 24 hours found the reaction to be complete with less than 5% of 4-methyl salicylic acid remaining. The reaction was cooled to 25°C and 2.26 L of water (7.8 ml/g) was added. The product then precipitated from the reaction. This mixture was left to stir at room temperature for 18 hours and was then cooled to 0°C and granulated for 1 hour. The recovered solid was filtered and washed with 0.23 L (0.8 ml/g) of isopropylacetate followed by 1.13 L (4 ml/g) of water. The damp cake was dried in a vacuum oven at 55°C for 18 hours. 299 g of the crude of *cis*-5-Fluoro-N-[4-(2-hydroxy-4-methylbenzamido)cyclohexyl]-2-(tetrahydro-thiopyran-4-yloxy)-nicotinamide was then obtained (recovery: 82.14%).

^{**1**}**HNMR (CDCl3, 400MHz)** δ: 1.60-2.10 (m, 10H), 2.30-2.50 (m, 5H), 2.70-2.94 (m, 4H), 4.06-4.34 (m, 2H), 5.46 (m, 1 H), 6.28 (m, 1 H), 6.68 (1 H, d), 6.80 (s, 1 H), 7.32 (d, 1 H), 8.00-8.18 (m, 2H), 8.28 (m, 1 H), 12.20 (brs, 1 H)

The crystalline form produced by the process described above has the following characteristics:

### PXRD

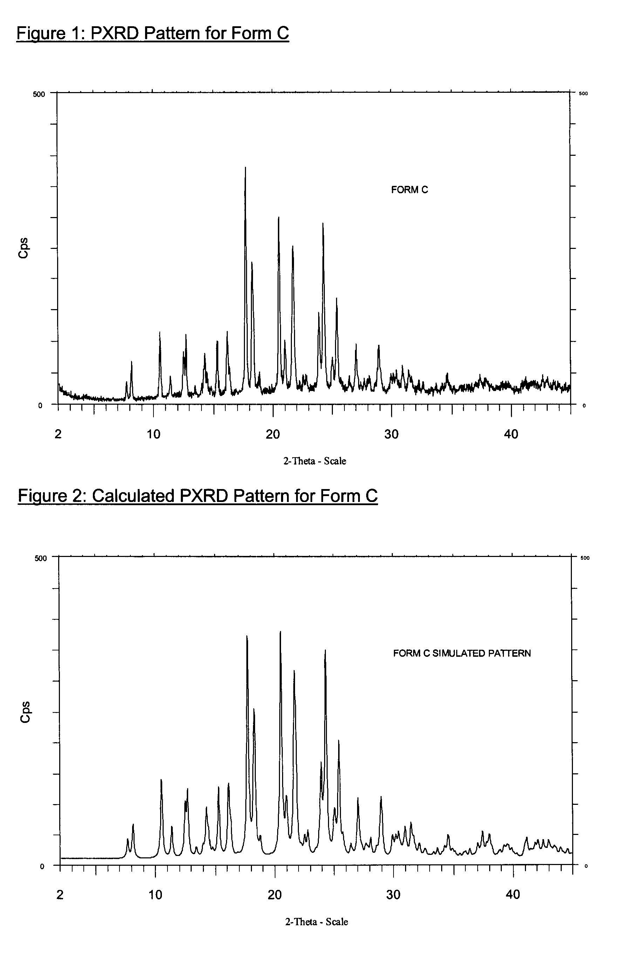

The PXRD pattern for Form C is shown in Figure 1. The main characteristic peaks are at 17.7, 18.3, 20.5, 21.7 and 24.3 degrees 2-theta and are further given in table 1.
The calculated PXRD pattern is illustrated in Figure 2. The main characteristic peaks from the calculated pattern for Form C are shown in table 2.

**Table 1: Characteristic PXRD Peaks for Form C**

| Angle 2-Theta (Degrees) | Intensity (%) | Angle 2-Theta (Degrees) | Intensity (%) |
|---|---|---|---|
| 8.1 | 17.6 | 20.5 | 79.0 |
| 10.5 | 30.0 | 21.0 | 26.4 |
| 12.5 | 19.3 | 21.7 | 66.7 |
| 12.7 | 28.9 | 23.9 | 38.0 |
| 14.2 | 21.0 | 24.3 | 76.3 |
| 15.3 | 26.3 | 25.0 | 19.5 |
| 16.1 | 30.2 | 25.4 | 44.1 |
| 16.3 | 16.1 | 27.0 | 24.9 |
| 17.7 | 100.0 | 28.9 | 24.5 |
| 18.3 | 59.5 | 30.9 | 16.0 |

**Table 2: Characteristic PXRD Peaks from calculated pattern for Form C**

| Angle 2-Theta (Degrees) | Intensity (%) | Angle 2-Theta (Degrees) | Intensity (%) |
|---|---|---|---|
| 8.1 | 17.6 | 20.5 | 100.0 |
| 10.5 | 36.9 | 21.0 | 30.1 |
| 11.3 | 16.6 | 21.7 | 83.3 |
| 12.5 | 27.7 | 22.8 | 15.0 |
| 12.7 | 33.7 | 23.9 | 44.4 |
| 14.2 | 25.1 | 24.3 | 92.0 |
| 14.4 | 18.1 | 25.0 | 24.7 |
| 15.3 | 33.6 | 25.4 | 53.7 |
| 16.1 | 35.4 | 27.0 | 28.8 |
| 16.2 | 23.4 | 29.0 | 29.5 |
| 17.7 | 98.1 | 31.0 | 16.5 |
| 18.3 | 67.4 | 31.5 | 18.4 |

### DSC

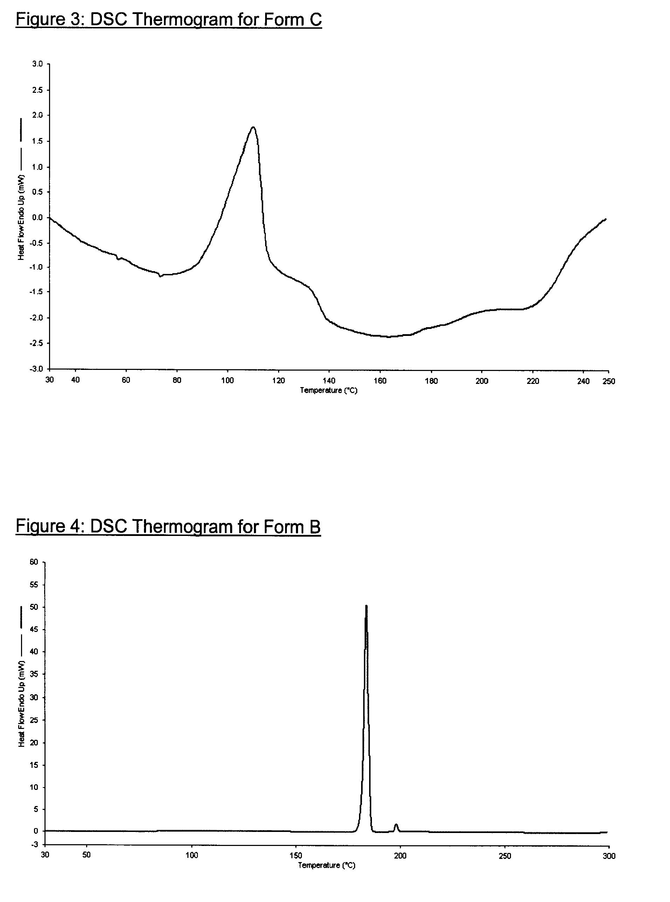

DSC analysis of a 3.050 mg sample of Form C was performed as described above. The DSC thermogram for Form C is shown in Figure 3. Form C shows an endothermic peak at 110°C ± 5°C. This is due to dehydration of Form C.

### Example 2: preparation of Form B (2^{nd} crystalline anhydrous form)

This crystalline form (so-called Form B) has the following characteristics:

### DSC:

A sample of 3.187 mg of Form B was analysed by DSC as described above. The DSC thermogram for Form B is shown in Figure 4. Form B shows a sharp endothermic peak at 184°C ± 2°C, with a smaller endothermic peak at 198°C ± 6°C. The peak at 184°C ± 2°C is due to melt of form B while the peak at 198°C ± 6°C is due to the melt of form A.

### PXRD:

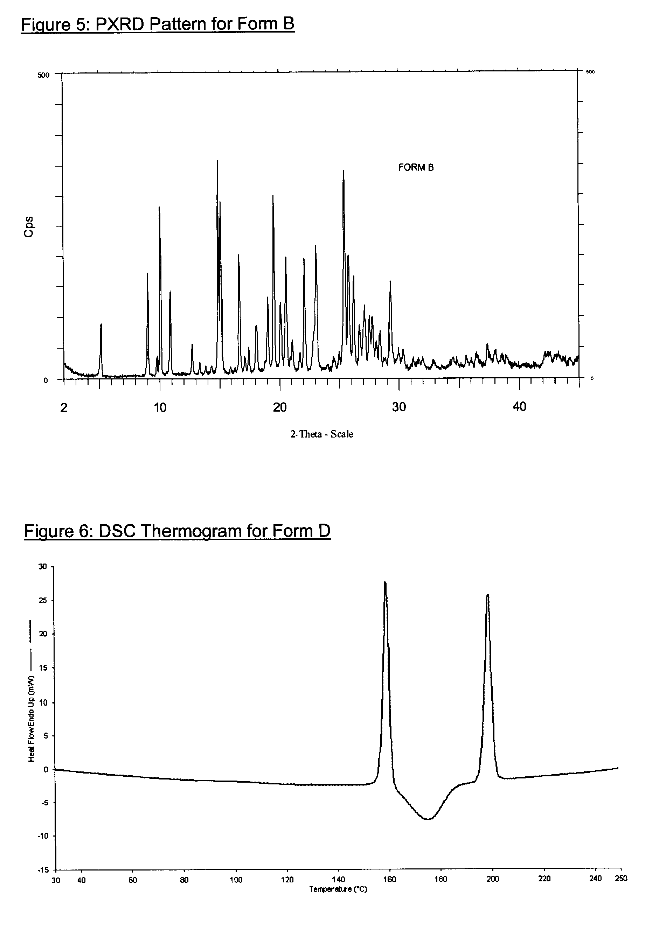

The PXRD pattern for Form B is shown in Figure 5. The main characteristic peaks are at 10.0, 14.9, 15.1, 19.5 and 25.4 degrees 2-theta and are further given in Table 3 below.

**Table 3: Characteristic PXRD Peaks for Form B**

| Angle 2-Theta (Degrees) | Intensity (%) | Angle 2-Theta (Degrees) | Intensity (%) |
|---|---|---|---|
| 5.0 | 24.3 | 22.1 | 54.5 |
| 9.0 | 47.8 | 22.9 | 21.8 |
| 10.0 | 78.5 | 23.1 | 60.4 |
| 10.8 | 39.6 | 25.4 | 95.1 |
| 12.6 | 15.6 | 25.8 | 56.0 |
| 14.9 | 100.0 | 26.2 | 45.9 |
| 15.1 | 80.9 | 26.7 | 23.3 |
| 16.6 | 56.2 | 27.1 | 32.7 |
| 18.0 | 23.4 | 27.5 | 27.9 |
| 19.0 | 36.4 | 27.8 | 27.5 |
| 19.5 | 83.7 | 28.1 | 16.8 |
| 20.1 | 34.1 | 28.4 | 20.0 |
| 20.5 | 55.2 | 29.3 | 43.9 |
| 21.1 | 17.1 | 37.4 | 15.1 |

### Example 3: Preparation of Form D (3^{rd} anhydrous crystalline form)

1.0 g (0.002 M) of Form C as obtained according to example 1 was charged to a reaction flask. 20 ml/g of MeCN was added and the mixture was heated to reflux (82°C). A solution was formed at reflux. Solution was left to cool to room temperature and solid re-precipitated. Reaction slurry was cooled to 0-5°C in an ice bath for one hour and was then filtered and washed with 2 ml/g of MeCN. 0.62 g of *cis*-5-Fluoro-N-[4-(2-hydroxy-4-methylbenzamido)cyclohexyl]-2-(tetrahydro-thiopyran-4-yloxy)-nicotinamide were then obtained (recovery: 62%) with a purity of 99.5%.

^{**1**}**HNMR (CDCl3, 400MHz)** δ: 1.60-2.10 (m, 10H), 2.30-2.50 (m, 5H), 2.70-2.94 (m, 4H), 4.06-4.34 (m, 2H), 5.46 (m, 1 H), 6.28 (m, 1 H), 6.68 (1 H, d), 6.80 (s, 1 H), 7.32 (d, 1 H), 8.00-8.18 (m, 2H), 8.28 (m, 1 H), 12.20 (brs, 1 H)

The crystalline form produced by the process described above (so-called Form D) has the following characteristics:

### DSC:

A sample of 3.147 mg of Form D was analysed by DSC as described above. The DSC thermogram for Form D is shown in Figure 6. Form D shows a sharp endothermic peak at 159°C ± 2°C, followed by an exothermic recrystallisation event at 175°C ± 2°C and a second endothermic peak at 199°C ± 6°C. The peak at 159°C ± 2°C is due to melt of form D while the peak at 199°C ± 6°C is due to the melt of form A.

### PXRD:

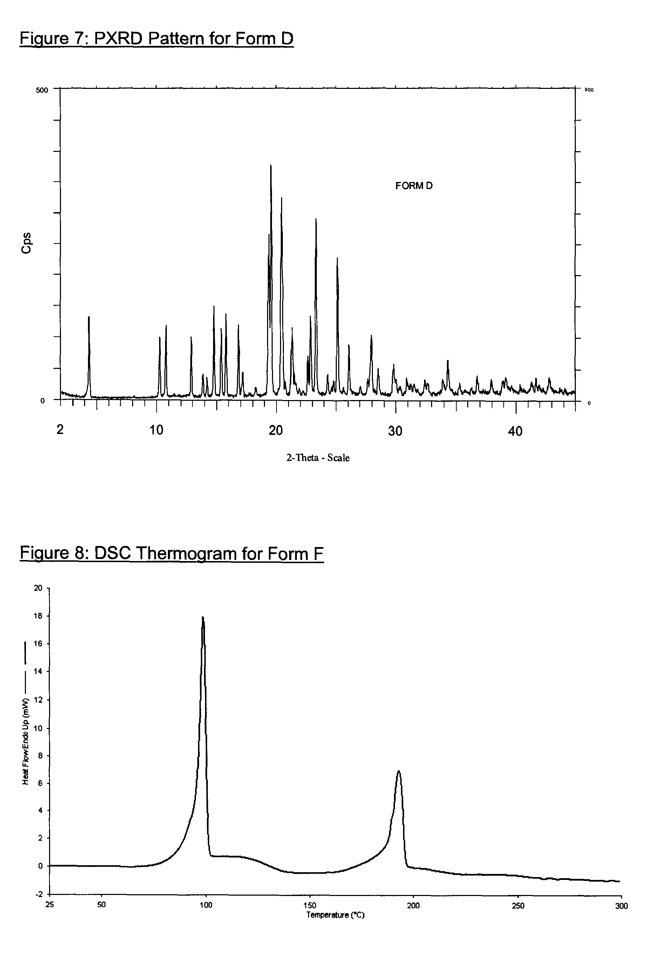

The PXRD pattern for Form D is shown in Figure 7. The main characteristic peaks are at 19.4, 19.6, 20.4, 23.3 and 25.1 degrees 2-theta and are further given in Table 4 below.

**Table 4: Characteristic PXRD Peaks for Form D**

| Angle 2-Theta (Degrees) | Intensity (%) | Angle 2-Theta (Degrees) | Intensity (%) |
|---|---|---|---|
| 4.3 | 34.8 | 21.2 | 23.1 |
| 10.2 | 26.2 | 21.3 | 30.3 |
| 10.7 | 31.3 | 22.6 | 18.4 |
| 12.9 | 26.6 | 22.8 | 35.3 |
| 14.8 | 39.5 | 23.3 | 77.4 |
| 15.3 | 29.9 | 25.1 | 60.5 |
| 15.7 | 36.1 | 26.1 | 23.2 |
| 16.8 | 31.6 | 27.9 | 27.2 |
| 19.4 | 70.4 | 29.8 | 15.3 |
| 19.6 | 100.0 | 34.3 | 16.9 |
| 20.4 | 85.9 | | |

### Example 4: Preparation of Form F (solvate crystalline form)

A suspension of 1.5 g of Form C as obtained according to example 1 above in 1.5 ml of dimethylformamide (DMF) was gradually warmed to 60°C. More DMF was slowly added until all the material had completely dissolved (approximately 3.4 ml to 4 ml was required). The clear solution was stirred for 30 minutes at 60°C and then gradually cooled to 20°C at a rate of 10°C/minute. Stirring was continued at this temperature for an additional 16 hours. The resulting thick suspension was centrifuged (3500 rpm for 15 minutes). The supernatant was decanted and the solid was dried under vacuum at room temperature until weight reduction was negligible.

The solvate crystalline form produced by the process described above (so-called Form F) has the following characteristics:

### DSC:

A sample of 3.024 mg of Form F was analysed by DSC as described above. The DSC thermogram for Form F is shown in Figure 8. Form F shows a sharp endothermic peak at 99°C ± 5°C, followed by a second endothermic peak at 193°C ± 6°C. The peak at 99°C ± 5°C is due to the desolvation of dimethylformamide (DMF) while the peak at 193°C ± 6°C is due to the melt of form A.

### PXRD:

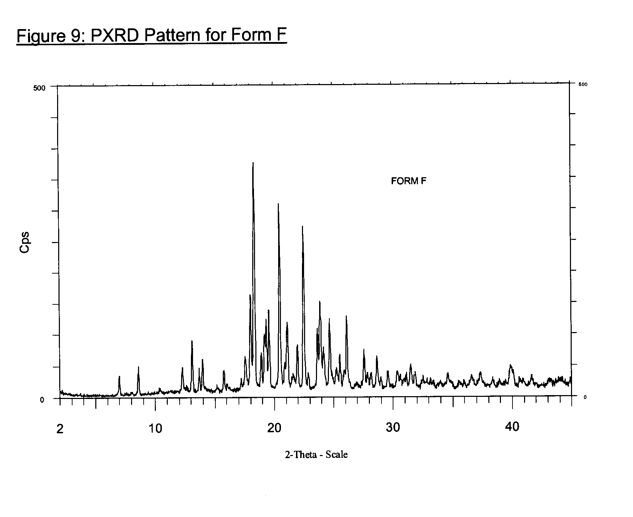

The PXRD pattern for Form F is shown in Figure 9. The main characteristic peaks are at 18.0, 18.4, 20.5, 22.5 and 23.9 degrees 2-theta and are further given in Table 5 below.

**Table 5: Characteristic PXRD Peaks for Form F**

| Angle 2-Theta (Degrees) | Intensity (%) | Angle 2-Theta (Degrees) | Intensity % |
|---|---|---|---|
| 13.1 | 23.6 | 22.0 | 21.8 |
| 14.0 | 16.0 | 22.5 | 72.6 |
| 17.6 | 16.0 | 23.7 | 28.6 |
| 18.0 | 43.4 | 23.9 | 40.4 |
| 18.4 | 100.0 | 24.2 | 21.0 |
| 18.9 | 18.6 | 24.7 | 32.9 |
| 19.2 | 27.7 | 25.5 | 17.9 |
| 19.3 | 31.9 | 26.1 | 34.1 |
| 19.6 | 36.6 | 27.6 | 19.9 |
| 20.5 | 82.5 | 28.7 | 17.0 |
| 21.1 | 31.3 | | |

### Preparation of cis-5-Fluoro-N-[4-(2-hydroxy-4-methylbenzamido)cyclohexyl]-2-(tetrahydro-thiopyran-4-yloxy)-nicotinamide according to PCT/IB04/002367

N-Methylmorpholine (11.16ml, 101.7mmol), 1-hydroxybenzotriazole hydate (7.49g, 55.5mmol) and 1-(3-dimethylaminopropyl)-3-ethylcarbodiimide hydrochloride (10.63g, 55.5mmol) were added portionwise to a suspension of the amine from preparation 18 (18g, 46.2mmol) in N,N-dimethylformamide (180ml). A solution of 4-Methyl salicylic acid (8.43g, 55.5mmol) in N,N-dimethylformamide (40ml) was added dropwise over 90 minutes, and once addition was complete, the reaction was stirred at room temperature for 72 hours. The mixture was concentrated under reduced pressure and the residue suspended in a mixture of tetrahydrofuran and 1 N sodium hydroxide solution, and the mixture stirred at room temperature for 1 hour. The tetrahydrofuran was removed *in vacuo* and the residual aqueous solution was diluted with water (750ml), and extracted with dichloromethane (2L in total). The combined organic solutions were washed with 2N hydrochloric acid (150ml), dried (MgSO₄) and evaporated under reduced pressure. The residue was suspended in methanol (250ml), the suspension stirred at room temperature for 18 hours. The resulting solid was filtered off, washed with methanol and dried *in vacuo* to give the title compound, 20.1g.

^{**1**}**H-NMR (CDCl**_{**3**}**, 400MHz)** δ: 1.71 (m, 2H), 1.81 (m, 2H), 1.88-2.06 (m, 6H), 2.33 (s, 3H), 2.40 (m, 2H), 2.77 (m, 2H), 2.84 (m, 2H), 4.15 (m, 1 H), 4.26 (m, 1 H), 5.47 (m, 1 H), 6.28 (m, 1 H), 6.67 (m, 1 H), 6.80 (s, 1H), 7.31 (d, 1 H), 8.07 (d, 1 H), 8.10 (d, 1 H), 8.29 (dd, 1 H), 12.30 (brs, 1 H).

A sample of syn-5-Fluoro-N-[4-(2-hydroxy-4-methyl-benzoylamino)-cyclohexyl]-2-(tetrahydro-thiopyran-4-yloxy)-nicotinamide that had been prepared using the process described in the above paragraph (ex. 63 of PCT/IB04/002367) was examined by PXRD and DSC according to the protocols previously described and was found to be in a crystalline anhydrous form (so-called "Form A") that is different from the solid forms as here above described (Form B, C, D and F).

Namely, Form A is characterized by a powder X-ray diffraction pattern, obtained using copper K-alpha₁ X-ray (wavelength = 1.54056 Angstroms), showing main peaks at 13.4, 18.1, 19.7, 20.5 and 22.6 degrees 2θ and by an endothermic peak at 195°C ± 6°C occurring during themal analysis using DSC.

### Preparation 2: Trans-N-tert-butyl (4-hydroxy-cyclohexyl)-carbamate

Trans-4-aminocyclohexanol (100g, 0.87mol) was added to acetonitrile (1 L), with stirring followed by di-*tert*-butyl dicarbonate (208g, 0.96mol) in portions over 1 hour. The reaction was stirred at room temperature for 18 hours, the resulting precipitate filtered off, and washed with ethyl acetate:hexane (1:3, 250ml), then hexane (250ml) and dried to afford the title compound as a white solid, 166.9g.

### Preparation 3: Trans-Methanesulphonic acid 4-tert-butoxycarbonylamino-cyclohexyl ester

A solution of mesyl chloride (122.4g, 1.07mol) in dichloromethane (400ml) was added dropwise over 45 minutes to an ice-cooled solution of the alcohol from preparation 2 (200g, 0.93mol) and triethylamine (112.8g, 1.115mol) in dichloromethane (1L). The reaction was stirred for 15 minutes, then allowed to warm to room temperature over 1 hour. The mixture was washed with water (3x1.5L), then stirred with silica (100ml, Merck 60H). This mixture was filtered and the filtrate concentrated under reduced pressure to approx quarter volume. Hexane (500ml) was added, the mixture cooled to 0°C, the resulting solid filtered off, dried and recrystallised from ethyl acetate to give the title compound, 221.1g.

### Preparation 4: syn-(4-Azido-cyclohexyl)-carbamic acid tert-butyl ester

Sodium azide (25.5g, 0.39mol) was added to a solution of the mesylate from preparation 3 (100g, 0.34mol) in N,N-dimethylformamide (500ml) and the reaction slowly warmed to 80°C, and stirred for a further 24 hours at this temperature. Ice/water (1L) was added slowly to the cooled reaction, and the resulting precipitate was filtered off, washed with water and dried. The solid was dissolved in ethyl acetate (200ml), the solution washed with water, dried (MgSO₄) and evaporated under reduced pressure. The residual solid was recrystallised from hexane to afford the title compound as a white solid, 50.8g.

### Preparation 5: Syn-tert-Butyl 4-aminocyclohexylcarbamate

5% Palladium on charcoal (5 g) was mixed with toluene (10 ml) and was added to the azide from preparation 4 (170 g, 0.71 mol) in methanol (400 ml). The mixture was hydrogenated (80 atmospheres) at room temperature for 18 hours and then filtered. The solvent was evaporated *in-vacuo* and the residue was triturated with ethyl acetate (50 ml) and then with hexane (200 ml). The solid obtained was isolated by filtration, dissolved in ethyl acetate (600 ml) and filtered through Celite® . The filtrate was concentrated *in-vacuo* to give a slush that was diluted with hexane (300 ml). The solid obtained was isolated by filtration and was washed with ethyl acetate in hexane (20:80). The mother liquors were combined and evaporated *in-vacuo*, the residue was purified by chromatography on silica gel using ethyl acetate and then methanol as eluant. The material obtained was crystallised from ethyl acetate and hexane and combined with the first crop to give the title compound as a white solid (76 g).

### Preparation 10: Syn-{4-[(2-Chloro-pyridine-3-carbonyl)-amino]-cyclohexyl}-carbamic acid tert-butyl ester

Oxalyl chloride (8ml, 90mmol) was added over 10 minutes to an ice-cooled suspension of 2-chloronicotinic acid (10g, 57 mmol) and N,N-dimethylformamide (5 drops) in dichloromethane (200ml). The suspension was then stirred at room temperature for 3 hours, and concentrated under reduced pressure. The residue was azeotroped with dichloromethane to give the intermediate acid chloride as a white solid. This was dissolved in dichloromethane (200ml), the solution cooled in a water bath, then N-diisopropylethylamine (20ml, 115mmol) and the amine from preparation 5 (13.4g, 62mmol) added. The reaction mixture was stirred for 18 hours, diluted with dichloromethane (100ml) and washed sequentially with 10% citric acid solution, saturated sodium bicarbonate solution (x2), water then brine. The organic solution was dried (MgSO₄) and evaporated under reduced pressure to afford the title compound in 97% yield.

### Preparation 14: Syn-(4-{[2-(Tetrahydrothiopyran-4-yloxy)-pyridine-3-carbonyl]-amino}-cyclohexyl)-carbamic acid tert-butyl ester

A mixture of the chloride from preparation 10 (1 g, 2.57mmol), tetrahydrothiopyran-4-ol (WO 94/14793, pg 77) (500mg, 4.23mmol) and cesium carbonate (1.4g, 4.23mmol) in acetonitrile (5ml) was stirred at reflux for 20 hours. The cooled mixture was partitioned between water (75ml) and ethyl acetate (75ml) and the layers separated. The organic phase was washed with water, 1 N HCl, saturated sodium bicarbonate solution and brine, then dried (MgSO₄) and evaporated under reduced pressure. The product was purified by column chromatography on silica gel using an elution gradient of ethyl acetate:pentane (5:95 to 70:30) to afford the title compound in 84 % yield.

### Preparation 18: Syn-N-(4-Amino-cyclohexyl)-2-(tetrahydrothiopyran-4-yloxy)-nicotinamide hydrochloride

4N Hydrochloric acid in dioxan (50ml) was added to a solution of the protected amine from preparation 14 (4.1g, 9.0mmol) in dichloromethane (10ml), and the reaction stirred at room temperature for 3 hours. The mixture was evaporated under reduced pressure, the residue suspended in ether and the suspension sonicated. The mixture was filtered, the solid dried at 50°C *in vacuo* to give the title compound in 96 % yield.

## Claims

1. crystalline forms of *cis*-5-Fluoro-N-[4-(2-hydroxy-4-methylbenzamido)cyclohexyl]-2-(tetrahydro-thiopyran-4-yloxy)-nicotinamide and salts thereof except Form A which is **characterized by** a powder X-ray diffraction pattern, obtained using copper K-alpha₁ X-ray (wavelength = 1.54056 Angstroms), showing main peaks at 13.4, 18.1, 19.7, 20.5 and 22.6 degrees 2θ.

2. Crystalline forms of *cis*-5-Fluoro-N-[4-(2-hydroxy-4-methylbenzamido)cyclohexyl]-2-(tetrahydro-thiopyran-4-yloxy)-nicotinamide and salts thereof according to claim 1 **characterized in that** they are anhydrous.

3. Anhydrous crystalline form according to claim 2, which is selected from Form B and Form D.

4. Anhydrous crystalline Form B according to claim 3, **characterized by** a powder X-ray diffraction pattern, obtained using copper K-alpha₁ X-ray (wavelength = 1.54056 Angstroms), showing main peaks at 10.0, 14.9, 15.1, 19.5 and 25.4 degrees 2θ.

5. Anhydrous crystalline Form D according to claim 3, **characterized by** a powder X-ray diffraction pattern, obtained using copper K-alpha₁ X-ray (wavelength = 1.54056 Angstroms), showing main peaks at 19.4, 19.6, 20.4, 23.3 and 25.1 degrees 2θ.

6. Crystalline forms of *cis*-5-Fluoro-N-[4-(2-hydroxy-4-methylbenzamido)cyclohexyl]-2-(tetrahydro-thiopyran-4-yloxy)-nicotinamide and salts thereof according to claim 1 which are monohydrates.

7. A monohydrate crystalline form according to claim 6, which is Form C.

8. monohydrate crystalline Form C according to claim 7, **characterized by** a powder X-ray diffraction pattern, obtained using copper K-alpha₁ X-ray (wavelength = 1.54056 Angstroms), showing main peaks at 17.7, 18.3, 20.5, 21.7 and 24.3 degrees 2θ.

9. Crystalline forms of cis-5-Fluoro-N-[4-(2-hydroxy-4-methylbenzamido)cyclohexyl]-2-(tetrahydro-thiopyran-4-yloxy)-nicotinamide and salts thereof according to claim 1 which are solvates.

10. A solvate crystalline form according to claim 9, which is Form F.

11. solvate crystalline Form F according to claim 10, **characterized by** a powder X-ray diffraction pattern, obtained using copper K-alpha₁ X-ray (wavelength = 1.54056 Angstroms), showing main peaks at 18.0, 18.4, 20.5, 22.5 and 23.9 degrees 2θ.

12. A pharmaceutical composition comprising a crystalline form of *cis*-5-Fluoro-N-[4-(2-hydroxy-4-methylbenzamido)cyclohexyl]-2-(tetrahydro-thiopyran-4-yloxy)-nicotinamide and salts thereof, as defined in any one of claims 1 to 11, together with a pharmaceutically acceptable excipient, diluent or carrier.

13. A crystalline form of cis-5-Fluoro-N-[4-(2-hydroxy-4-methylbenzamido)cyclohexyl]-2-(tetrahydro-thiopyran-4-yloxy)-nicotinamide and salts thereof, as defined in any one of claims 1 to 11, for use as a medicament.

14. The use of a crystalline form of cis-5-Fluoro-N-[4-(2-hydroxy-4-methylbenzamido)cyclohexyl]-2-(tetrahydro-thiopyran-4-yloxy)-nicotinamide and salts thereof, as defined in any one of claims 1 to 11, for the manufacture of a medicament having a PDE4 inhibitory activity.

15. The use of a crystalline form of cis-5-Fluoro-N-[4-(2-hydroxy-4-methylbenzamido)cyclohexyl]-2-(tetrahydro-thiopyran-4-yloxy)-nicotinamide and salts thereof, as defined in any one of claims 1 to 11, for the manufacture of a medicament for the treatment of an inflammatory or respiratory disease.

16. The use according to claim 15 wherein said disease is selected from the group consisting of adult respiratory distress syndrome (ARDS), bronchitis, chronic bronchitis, chronic obstructive pulmonary disease (COPD), cystic fibrosis, asthma, emphysema, bronchiectasis, chronic sinusitis, rhinitis, inflammatory bowel diseases (IBD) such as Crohn's disease, ileitis, collagenous colitis, colitis polyposa, transmural colitis and ulcerative colitis.

17. A method of treatment of a mammal, including a human being, with a PDE4 inhibitor including treating said mammal with an effective amount of a crystalline form of *cis*-5-Fluoro-N-[4-(2-hydroxy-4-methylbenzamido)cyclohexyl]-2-(tetrahydro-thiopyran-4-yloxy)-nicotinamide and salts thereof, as defined in any one of claims 1 to 11.

18. A method of treatment of a mammal, including a human being, to treat an inflammatory or respiratory disease including treating said mammal with an effective amount of a crystalline form of *cis*-5-Fluoro-N-[4-(2-hydroxy-4-methylbenzamido)cyclohexyl]-2-(tetrahydro-thiopyran-4-yloxy)-nicotinamide and salts thereof, as claimed in any one of claims 1 to 11.

19. A method according to claim 18, wherein said disease is selected from the group consisting of adult respiratory distress syndrome (ARDS), bronchitis, chronic bronchitis, chronic obstructive pulmonary disease (COPD), cystic fibrosis, asthma, emphysema, bronchiectasis, chronic sinusitis, rhinitis, inflammatory bowel diseases (IBD) such as Crohn's disease, ileitis, collagenous colitis, colitis polyposa, transmural colitis and ulcerative colitis.
